# EUROPEAN PATENT SPECIFICATION

(11) **EP 1 694 229 B1**
(45) Date of publication and mention of the grant of the patent: **28.07.2010**
(21) Application number: 04806337.4
(22) Date of filing: 14.12.2004
(51) Int. Cl.: A61B 17/70

(54) **DEVICE FOR OSTEOSYNTHESIS OF THE SPINE**
VORRICHTUNG FÜR DIE OSTEOSYNTHESE DER WIRBELSÄULE
DISPOSITIF D'OSTEOSYNTHESE DE LA COLONNE VERTEBRALE

(30) Priority: 17.12.2003 FR 0314835
(43) Date of publication of application: 30.08.2006
(73) Proprietor: Warsaw Orthopedic, Inc., Warsaw, IN 46581 (US)
(72) Inventor: VARGA, Peter, H-1132 Budapest (HU); GOURNAY, José, 01170 GEX (FR); MAXY, Philippe, F-77410 CLAYE SOUILLY (FR)
(74) Representative: Neyret, Daniel Jean Marie
(86) International application number: PCT/IB2004/004104
(87) International publication number: WO 2005/058173

(56) References cited:
- WO-A-02/085217
- DE-A- 10 117 426
- FR-A- 2 720 261
- FR-A- 2 734 471
- GB-A- 2 294 394
- US-A- 5 702 395
- PATENT ABSTRACTS OF JAPAN vol. 1997, no. 04, 30 April 1997 (1997-04-30) & JP 08 336548 A (MIZUHO IKA KOGYO KK), 24 December 1996 (1996-12-24)

## Description

The invention concerns the field of devices for osteosynthesis of the spine.

In order to correct deformations of the spine such as scoliosis, spondylolisthesis, lordosis or kyphosis, it is known to use devices comprising one or more rods made of a metal, for example steel, which extend over at least part of the length of the spine and are made integral with one or two rows of anchoring elements (or implants) fitted on the spine. Such devices are described in the document US-A-5,005,562, for example.

These anchoring elements are often formed by screws which are implanted in a vertebra, or by hooks which are assigned an anchoring point on the lamina, pedicle or transverse process of a vertebra.

The head of these anchoring elements is designed to support at least one U-shaped channel which is perpendicular to the axis of the element and in which a rod is inserted. The U-shaped channel is delimited by two side branches. On their surface facing into the channel, these side branches in most cases have threads constituting an internal threading arrangement for insertion and immobilizing of a plug of generally cylindrical shape. This plug, when it is tightened, has the function of pressing the rod against the bottom of the channel and of keeping the rod there after the device has been fitted.

When the corrective device is being put in place, the rod is inserted into the U-shaped channels of the heads of the anchoring elements which have been fixed beforehand on the vertebrae, and the plugs are then screwed into the channels by means of a suitable screwdriver.

It is also known for the rod to be inserted not into the heads of the anchoring elements, but instead into connectors which are themselves mounted on the heads of the anchoring elements. What will be discussed below concerning the problems encountered when inserting the rod into the heads of the anchoring elements applies similarly to the case where the rod has to be inserted into connectors positioned beforehand on the heads of the anchoring elements.

In cases where it is necessary to correct a severe deformation of the spine, quite considerable mechanical stresses, for example in flexion and/or in rotation, have to be imposed on the rod when introducing it into the channels of the heads of the anchoring elements. In the zones where the deformation is at its maximum, the rod is at a distance from the implant, and it is necessary to use a heavy and bulky instrument to bring it between the branches of the U-shaped channel. This manoeuvre increases the operating time and sometimes necessitates an extension of the incisions made on the patient. The relatively high rigidity of the rod makes it difficult to deform in order to insert it into the heads of the anchoring elements. Moreover, the stresses generated upon introduction of the rod are directly transmitted to the bone-anchoring elements, thus compromising their long-term stability.

It has been proposed in documents GB-A-2 294 394, WO-A-02/41 797 and WO-A-02/085 217 to replace the single rod of large diameter in these devices with a plurality of rods of small diameter which the surgeon inserts one after another into channels defined by the heads of the anchoring elements. A threaded plug holds the rods in their seat. These rods are inserted either longitudinally (GB-A-2 294 394 and WO-A-02/085 217) or laterally (WO-1-02/41 797) into the heads of the anchoring elements. On account of the small diameter of each rod, it is easier for the surgeon to shape them so as to be able to insert them into the heads of the anchoring elements, and experience has shown that the cumulative effects of these multiple rods of small diameter, pressed against one another inside the heads by the plugs, are comparable to those of the customary single rods of large diameter.

Document DE 101 17426 discloses the technical features defined in the preamble of claim 1.

The object of the invention is to propose a variant of these devices which has improved properties.

To this end, the subject of the invention is a device for osteosynthesis of the spine, of the type comprising a plurality of bone-anchoring elements, a plurality of longitudinal elements, and connection means for connecting said longitudinal elements to said bone-anchoring elements, characterized in that at least one of the longitudinal elements is formed by a plurality of independent rods, and in that said connection means comprise at least one seat with a width substantially equal to the diameter of each rod, in which seat said rods are stacked so as to form a column.

At least some of said connection means can comprise the heads of the bone-anchoring elements.

At least some of said connection means can comprise connectors formed integrally with said bone-anchoring elements.

The device can comprise at least two longitudinal elements, each formed by a plurality of rods, said connection means comprising seats with a width substantially equal to the diameter of each rod, in a number equal to the number of longitudinal elements.

Said connection means can comprise at least one lateral opening for the introduction of said rods into said seats.

Said lateral opening can have a width 0.5 to 2 mm greater than the diameter of each of the rods.

Said seats can have different depths.

The device can comprise at least one longitudinal element formed by a single rod of relatively large diameter and at least one longitudinal element formed by a plurality of rods of relatively small diameter, and said connection means comprise seats with widths substantially equal to the diameter of the rods which they contain.

The connection means can comprise at least one plug which has an outer thread and which is inserted into a seat having a corresponding thread, and said plug, when it is tightened, holds at least one of the longitudinal elements in its seat.

Said plug is made up of an upper part having said outer thread, and of a lower part formed by a plate which is articulated on said upper part via a ball.

Said seats can be formed by recesses made on the bottom of the head of the bone-anchoring element, by a threaded protrusion made on the bottom of the head of the bone-anchoring element, and by the lower face of a nut borne by the protrusion, said nut allowing, after having been tightened, to keep the longitudinal elements in said seats.

Said protrusion can bear a cut which allows breaking it after the nut has been tightened.

As will have been understood, the invention lies in conferring upon the heads of the anchoring elements, or upon the connectors in which the multiple rods of small diameter are inserted, a configuration which makes it possible to form stacks of rods arranged in columns. In the prior art, the rods were grouped in the heads of the anchoring elements in a more or less "anarchic" manner. This arrangement of the rods in columns affords a number of advantages over and above those of the known devices.

The invention will be better understood on reading the description which is set out below and in which reference is made to the following attached figures:
- Figure 1 showing a perspective view of a first embodiment of the invention;
- Figure 2 showing a perspective view of a second embodiment of the invention;
- Figure 3 showing a cross section through an example of a threaded plug which can be used in the context of the invention;
- Figure 4 showing a perspective view of a third embodiment of the invention.
- Figure 5 showing a perspective view of a fourth embodiment of the invention.

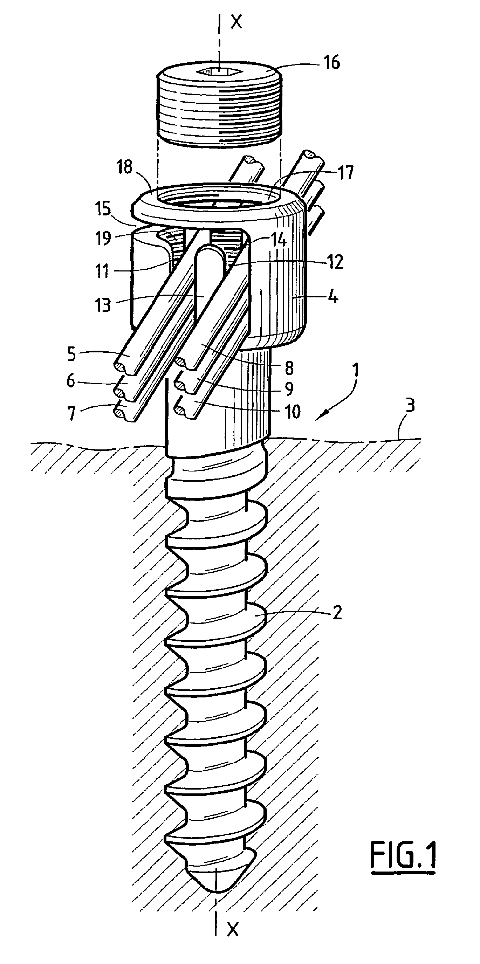

Figure 1 shows a bone-anchoring screw 1 of a device for correcting deformations of the spine in accordance with a first embodiment of the invention. This screw 1 has, as is customary, an externally threaded shank 2 permitting fixation of the screw 1 in a vertebra whose contour 3 is indicated by a broken line.

The screw 1 also comprises a head 4 into which independent metal rods 5, 6, 7, 8, 9, 10 of relatively small diameter, for example of the order of 1.3 to 3 mm, are inserted. These rods 5-10 constitute the longitudinal element extending along that part of the spine whose curvature is to be corrected, and for this purpose they pass through the heads of other screws which are similar to the screw 1 and are implanted in the vertebrae of the zone of the spine to be treated.

According to the invention, these rods 5-10 are not piled inside the head 4 in a random manner, but are instead disposed in an ordered way in two columns oriented in a direction parallel to the longitudinal axis X-X of the screw 1.

For this purpose, instead of a single U-shaped channel of wide cross section as in the prior art, two channels 11, 12 of U-shaped cross section are formed in the head 4, these channels 11, 12 having a small width substantially equal to the diameter of each of the rods 5-10. The channels 11, 12 are defined by means of two partitions 13, 14 which divide in two the openings formed in the side wall of the head 4 for the passage of the rods 5-10. These rods 5-10 are introduced into the head 4 laterally, through an opening 15 formed in the wall of the head 4 and above the channels 11, 12. The surgeon inserts the rod 5-10, which he wishes to fit, horizontally into this opening 15, then he orients the rod 5-10 in one or other of the channels 11, 12. He then repeats the procedure for each of the rods 5-10 of the corrective device. When all the rods 5-10 are in place, the surgeon immobilizes them by inserting, in a known manner, an externally threaded plug 16 into an orifice 17 formed on the upper face 18 of the head 4. For this purpose, the inside wall of the head 4 comprises a thread 19.

The width of the opening 15 is preferably only a little greater than the diameter of the rods 5-10 (for example 0.5 to 2 mm greater than this diameter) so as to make it difficult for the rods 5-10 to spontaneously exit the head 4 between their being put in place and the plug 16 being tightened.

In an alternative, the upper face 18 of the head 4 could be joined to the rest of the head 4 only at the level of the partitions 13, 14. This would then give two openings comparable to the opening 15 and facing each other, each of them being able to guide the rods 5-10 to one of the channels 11, 12.

Compared to the known devices where the rods are arranged in the heads of the screws in no particular order, the device according to the invention gives the surgeon more possibility of precisely adapting the properties of the corrective device as a function of the specific needs of the patient.

The number of rods 5-10 stacked in columns in the vertical direction (three per column in the example shown, although there could be more or fewer than three) determines the rigidity of the assembly in flexion and in extension. The number of channels 11, 12 (two in the example shown) determines the rigidity of the assembly in lateral flexion and in torsion. By acting on these parameters, and on the materials and dimensions of the rods 5-10, the surgeon can control at will the rigidity of the assembly in all spatial directions.

Moreover, the strictly ordered arrangement of the rods 5-10 inside the head 4 of the screw 1 permits exact control of the tightening stresses exerted by the plugs 16 (or any other device which would have an equivalent function) on the rods 5-10. In the devices of the prior art, in which the rods are piled in a random manner in the heads of the screws, the quality of the hold of each rod depends on its position in the pile and can vary greatly according to this position. For some rods, this hold may be insufficient to the point of allowing migration of the rods relative to one another. By contrast, the invention guarantees the rods 5-10 an at all times secure hold in the transverse direction, on account of the fact that no movement of the rods 5-10 is permitted in this direction.

In the longitudinal direction, the quality of the hold of the rods 5-10 depends only on the degree of tightening of the plug 16, which exerts stresses equally distributed on all the stacks of rods 5-7, 8-10. Also, because each stack of rods 5-7, 8-10 is oriented very substantially along the longitudinal axis of the plug 16, the stresses exerted on one rod are in principle transmitted identically to the rod below it, hence with maximum reliability.

In an alternative (not shown), it is possible to provide, on the periphery of the outer wall of the head 4, an indent which, after the plug 16 has been tightened, makes it possible to separate the upper part of the head 4 from the rest of the head 4 by folding and tearing at the level of the indent, in order thereby to reduce the size of the head 4. Such an indent with this function is known in the prior art on heads of conventional anchoring elements (see document WO 0200125).

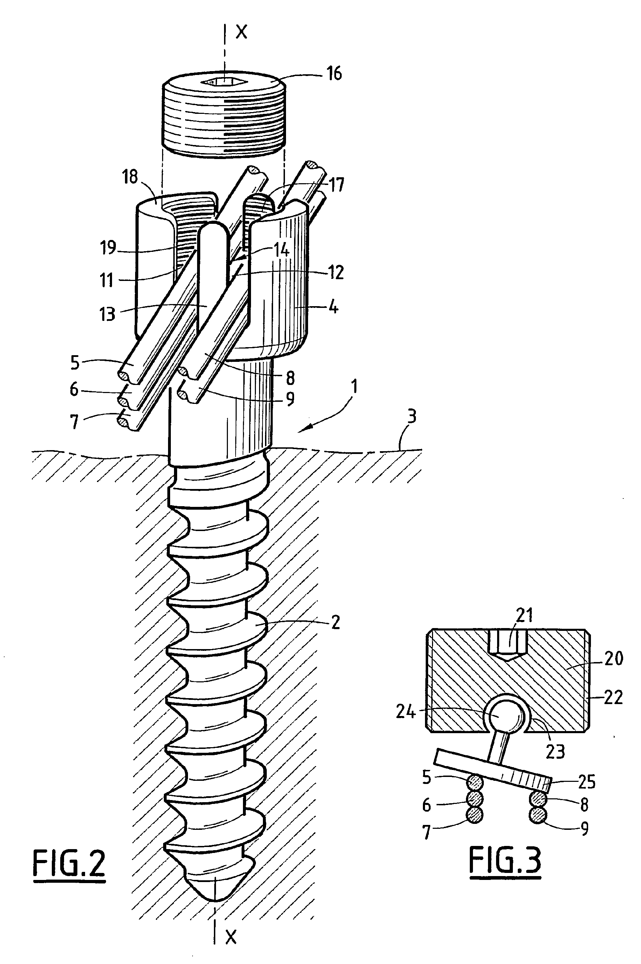

Figure 2 shows a variant of the invention which differs from the embodiment in Figure 1 in the following two aspects (the elements common to both embodiments are designated by the same reference numbers).

Firstly, the two channels 11, 12 in which the rods are stacked each have a different depth: the left-hand channel 11 can contain three stacked rods 5, 6, 7, whereas the right-hand channel 12, which is less deep, can contain two stacked rods 8, 9. This characteristic gives the surgeon additional freedom to adjust the properties of the corrective device. The lesser depth of the right-hand channel 12 means that the stack of rods 8, 9 it contains can have its upper surface at the same level as the stack of rods 5, 6, 7 in the left-hand channel 11. The lower face of the plug 16 can then act in the same way on both stacks.

Secondly, the rods 5-9 are introduced into the head not laterally, but longitudinally with respect to the axis of the screw 1, and through the same opening 17 which then serves for insertion of the threaded plug 16. The design of the head 4 of the screw 1 is thereby simplified. However, this can sometimes make it more difficult to introduce the rods 5-9 and especially hold them in the head 4 until the plug 16 has been put in place. Moreover, the configuration in Figure 1 affords the advantage of making it possible to place the plug 16 on the head 4 in the untightened state even before introduction of the rods 5-10 into the head 4. Assembly of the device is thus made faster and easier.

It must be understood that channels 11, 12 of different depths can also be provided on a screw 1 for lateral insertion of the rods, such as in Figure 1.

Another possible solution, in particular if one wishes to use a different number of rods in each channel, consists in not necessarily giving the channels 11, 12 different depths, but instead in using a plug of the type shown in Figure 3. It is made up of an upper part 20 with, as is customary, a recess 21 for insertion of a screwdriver on its upper face and a thread 22 on its side wall. On its lower face, the upper part 20 has a seat 23 for receiving a ball 24 attached to a preferably circular plate 25. The plate 25 can tilt in all spatial directions on account of the articulation of the ball 24 in the seat 23. In this way, as is shown diagrammatically, when the two stacks of rods 5, 6, 7 and 8, 9 do not have the same height, on account of the fact that the number of rods is not the same in the two channels 11, 12, or when the totals of the diameters of the rods present in identical numbers in each channel 11, 12 are not absolutely equal on account of the production tolerances of the rods 5-10, this plug nevertheless has the possibility, by way of the articulated plate 25, of exerting the same pressure on the two stacks 5, 6, 7 and 8, 9 or 5, 6, 7 and 8, 9, 10.

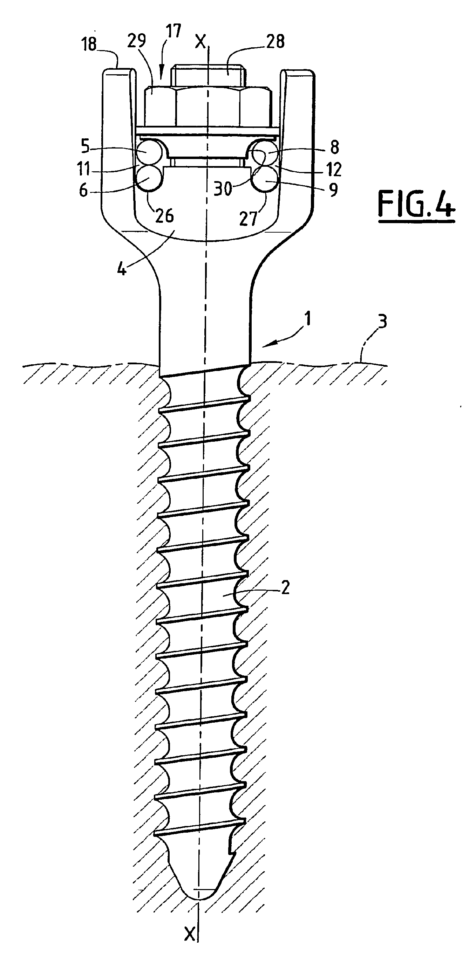

The embodiment shown in Figure 4 differs from the preceding one in that the means which define the seats in which the rods 5, 6, 8, 9 are stacked in columns do not have partitions 13, 14. But they have recesses 26, 27 made in the bottom of the head 4, in which the lower rod 6, 9 of a stack rests. They also have, made in the bottom of the head 4, a threaded protrusion 28 which bears the plug which performs the tightening of the rods 5, 6, 8, 9 in the head 4. This plug is a nut 29, the lower face 30 of which is formed so as to exert a pressure on the rods 5, 6, 8, 9 when the nut is tightened. Preferably, this configuration also ensures a lateral containment of at least the upper rod 5, 8 of each stack, for example, as shown, thaks to an enveloping curvature of the lower face 30 of the nut 29. It must be noticed that in this embodiment, it is no more necessary to make a thread on the inside wall of the head 4 for insertion and fixation of the plug, since the threaded protrusion 28 performs these functions.

Initially, the threaded protrusion 28 may be longer than what is shown, in order to make it significantly protrude from the upper face 18 of the head 4. So, it becomes possible to insert the nut 29 on the protrusion 28 before the rods 5, 6, 8, 9 are inserted into the head 4. It is , so, possible to obtain the advantages which have been cited for the embodiment shown in Figure 1. Moreover, the space which must be kept, before the nut 29 is tightened, between the lower face 30 of the nut 29 and the upper face 18 of the head 4, is functionally similar to the opening 15 of the embodiment shown in Figure 1. The nut 29, not tightened, yet, is so used as a guide when the rods 5, 6, 8, 9 are inserted into the head 4, and makes it more difficult for the rods 5, 6, 8, 9 to escape before they are locked. After the nut 29 has been tightened, the protrusion 28 can be broken along a cut made on its periphery, so as to give it its final length.

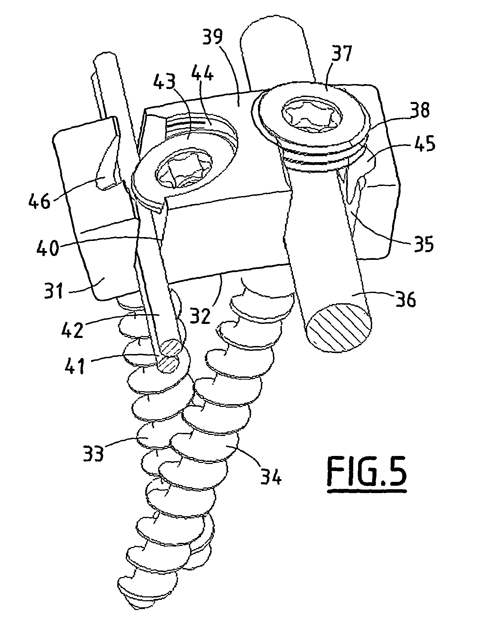

Another example of a device for correcting deformations of the spine in accordance with the invention is shown in Figure 5. Whereas the devices in Figures 1 and 2 comprised bone-anchoring screws 1 whose heads themselves were designed to receive stacks of rods 5-10 in columns, in this embodiment the element configured according to the invention is a connector 31. In the example shown, this is designed to be implanted on the anterior face of a vertebra (not shown), with its lower face 32 resting on the vertebra and substantially matching the shape of the latter. The connector 31 is held on the vertebra by means of two bone-anchoring screws 33, 34 which pass through orifices (not shown in Figure 5) formed on its lower face 32. It must be understood that it would also be possible to use a connector which did not rest on the vertebra but instead on the upper part of an anchoring element. Likewise, it would be possible to use just one of the bone-anchoring screws 33, 34. This would permit use of such a connector by the posterior approach, whereas the variant shown here applies to use by the anterior approach.

In the example shown, the two screws 33, 34 have convergent oblique orientations in order to permit a better quality of anchoring, but this characteristic is not obligatory.

The connector 31 comprises two channels which receive rods of the spinal osteosynthesis device, of which the connector 31 forms part. In the example shown, a first channel 35 is dimensioned in such a way as to receive a single rod 36 of relatively large diameter (4 to 7 mm for example) of the type used on the most common devices for osteosynthesis of the spine. An externally threaded plug 37, placed in a seat 38 with threaded walls opening out on the upper face 39 of the connector 31, holds the rod 36 in its seat 35. According to the invention, a second channel 40 is dimensioned in such a way as to receive an ordered stack of rods 41, 42 of relatively small diameter, of the type previously described (two rods 41, 42 are used in the example shown, but there could be more of them, depending on the choice made by the surgeon, the main point being that the connector 31 affords the possibility of forming a stack of rods 41, 42 of small diameter in a column formation). An externally threaded plug 43, placed in a seat 44 with a threaded wall opening out on the upper face 39 of the connector 31, holds the rods 41, 42 in their seat 40.

It will be noted, in the example shown, that two notches 45, 46 are present on the upper face 39 and the walls of the channels 35, 40, the function of these notches being to permit insertion of the screwdriver with which the screws 33, 34 can be tightened on the vertebra, before the rods 36, 41, 42 are placed in their respective seats 35, 40.

By combining the use of a rod 36 of relatively large diameter and a stack of rods 41, 42 of small diameter, which are ordered in a column, it is possible to benefit from the advantages of the invention when inserting the rods 41, 42 of small diameter into the connector 31, this insertion generally being done after that of the rod 36 of large diameter.

As alternatives, provision could be made for the connector 31 to comprise more than one channel 35 for rods of large diameter and/or more than one channel 405 in which to stack rods of small diameter.

This use of at least one rod 36 of large diameter and of at least one stack of rods 41, 42 of small diameter is not adapted exclusively to the use of a connector 31. The head of a bone-anchoring screw could also be designed in such a way as to permit this use.

Conversely, the designs of the heads of bone-anchoring screws given as examples in Figures 1 and 2 can easily be transposed to connectors.

In the configurations which have been described and shown, the longitudinal axes of the channels where the rods of small diameter are stacked are parallel to the longitudinal axis of the bone-anchoring element or of the connector. However, there may be an angle between these two axes (for example of up to 45°).

The materials from which the rods 5-10, 36, 41, 42 can be made include all the known alloys for similar uses, in particular stainless steel, titanium and its alloys, and shape-memory materials of the NiTi type.

It should be noted that the invention makes it possible to use materials having properties of elongation of the order of 5 to 8%, hence greater than for the materials which are customarily used to form the single rods of large diameter in the existing devices.

The reason is that, in the event of a flexion movement of the spine, the upper rods work in traction while the lower rods work in compression. If the material of the rods has a low possibility of elongation, there is a tendency toward a relative displacement of the rods 5-10, 41, 42 and this can cause friction, and hence wear and corrosion of the rods 5-10, 41, 42. Providing rods 5-10, 41, 42 with quite considerable possibilities of elongation makes it possible to absorb some of the compression and distraction exerted on the rods 5-10, 41, 42. Examples of such materials which may be mentioned are various metal materials, in particular shape-memory alloys such as Nitinol (alloy of nickel and titanium), or elastomers (polyethylene, silicone) with capacities of elongation and compression greater than those of the metal materials. It is also possible to conceive using composite rods, where one or more metal filaments are embedded in elastomer.

The invention can also be used for polyaxial screws and connectors, for which the respective angular orientations of the rod-bearing part and of the bone-anchoring part or portion can be chosen, thanks, for example, to a spherical seat on which these elements are articulated.

Instead of screws, the bone-anchoring elements can also be hooks which are assigned anchoring points on the lamina, pedicle or transverse process of the vertebrae.

## Claims

1. Device for osteosynthesis of the spine, of the type comprising a plurality of bone-anchoring elements (2; 33, 34), a plurality of longitudinal elements (5-10; 36, 41, 42), and connection means (4; 31) for connecting said longitudinal elements (5-10; 36, 41, 42) to said bone-anchoring elements (2; 33, 34), wherein at least one of the longitudinal elements is formed by a plurality of independent rods (5-7; 8-10; 41, 42), and in that said connection means (4; 31) comprise at least one seat (11, 12; 40) with a width substantially equal to the diameter of each rod (5-10; 41, 42), **characterized in that** said rods (5-10; 41,42) are stacked in said seat so as to form a column in cross-section.

2. Device according to Claim 1, **characterized in that** at least some of said connection means comprise the heads (4) of the bone-anchoring elements (2).

3. Device according to Claim 1 or 2, **characterized in that** at least some of said connection means comprise connectors (31) formed integrally with said bone-anchoring elements (33, 34).

4. Device according to one of Claims 1 to 3, **characterized in that** it comprises at least two longitudinal elements, each formed by a plurality of rods (5-7, 8-10), said connection means comprising seats (11, 12) with a width substantially equal to the diameter of each rod (5-7, 8-10), in a number equal to the number of longitudinal elements.

5. Device according to one of Claims 1 to 4, **characterized in that** said connection means comprise at least one lateral opening (15) for the introduction of said rods (5-10) into said seats (11, 12).

6. Device according to Claim 5, **characterized in that** said lateral opening (15) has a width 0.5 to 2 mm greater than the diameter of each of the rods (5-10).

7. Device according to one of Claims 4 to 6, **characterized in that** said seats (11, 12) have different depths.

8. Device according to one of Claims 1 to 7, **characterized in that** it comprises at least one longitudinal element formed by a single rod (36) of relatively large diameter and at least one longitudinal element formed by a plurality of rods (41, 42) of relatively small diameter, and **in that** said connection means (31) comprise seats (35, 40) with widths substantially equal to the diameter of the rods which they contain.

9. Device according to one of Claims 1 to 8, **characterized in that** the connection means comprise at least one plug (16) which has an outer thread (22) and which is inserted into a seat (17) having a corresponding thread (19), and said plug (16), when it is tightened, holds at least one of the longitudinal elements in its seat.

10. Device according to Claim 9, **characterized in that** said plug (16) is made up of an upper part (20) having said outer thread (22), and of a lower part formed by a plate (25) which is articulated on said upper part via a ball (24).

11. Device according to one of claims 1 to 4, **characterized in that** said seats (11, 12) can be formed by recesses (26, 27) made on the bottom of the head (4) of the bone-anchoring element, by a threaded protrusion (28) made on the bottom of the head (4) of the bone-anchoring element, and by the lower face (30) of a nut (29) borne by the protrusion (28), said nut (29) allowing, after having been tightened, to keep the longitudinal elements in said seats (11, 12).

12. Device according to claim 11, **characterized in that** said protrusion (28) can bear a cut which allows breaking it after the nut (29) has been tightened.

## Patentansprüche

1. Vorrichtung zur Osteosynthese der Spina des Typs, der mehrere Knochenverankerungselemente (2; 33, 34), mehrere Längselemente (5 - 10; 36, 41, 42) und ein Verbindungsmittel (4; 31) zum Verbinden der Längselemente (5 - 10; 36, 41, 42) mit den Knochenverankerungselementen (2; 33; 34) umfasst, wobei mindestens eines der Längselemente durch mehrere unabhängige Stäbe (5 - 7; 8 - 10; 41, 42) gebildet ist, und wobei das Verbindungsmittel (4; 31) mindestens einen Sitz (11, 12; 40) mit einer Breite umfasst, die im Wesentlichen gleich dem Durchmesser jedes Stabs (5 - 10; 41, 42) ist, **dadurch gekennzeichnet, dass** die Stäbe (5 - 10; 41, 42) in dem Sitz gestapelt sind, um im Querschnitt eine Säule zu bilden.

2. Vorrichtung nach Anspruch 1,
**dadurch gekennzeichnet, dass** mindestens ein Teil des Verbindungsmittels die Köpfe (4) der Knochenverankerungselemente (2) umfasst.

3. Vorrichtung nach Anspruch 1 oder 2,
**dadurch gekennzeichnet, dass** mindestens ein Teil des Verbindungsmittels Verbinder (31) umfasst, die einstückig mit den Knochenverankerungselementen (33, 34) ausgebildet sind.

4. Vorrichtung nach einem der Ansprüche 1 bis 3,
**dadurch gekennzeichnet, dass** sie mindestens zwei Längselemente umfasst, wobei jedes durch mehrere Stäbe (5 - 7,8 - 10) gebildet ist, wobei das Verbindungsmittel Sitze (11, 12) mit einer Breite, die im Wesentlichen gleich dem Durchmesser jedes Stabs (5 - 7, 8 - 10) ist, in einer Anzahl, die gleich der Anzahl der Längselemente ist, umfasst.

5. Vorrichtung nach einem der Ansprüche 1 bis 4,
**dadurch gekennzeichnet, dass** das Verbindungsmittel mindestens eine laterale Öffnung (15) für die Einführung der Stäbe (5 - 10) in die Sitze (11, 12) umfasst.

6. Vorrichtung nach Anspruch 5,
**dadurch gekennzeichnet, dass** die laterale Öffnung (15) eine Breite aufweist, die um 0,5 bis 2 mm größer als der Durchmesser jedes der Stäbe (5 - 10) ist.

7. Vorrichtung nach einem der Ansprüche 4 bis 6,
**dadurch gekennzeichnet, dass** die Sitze (11, 12) unterschiedliche Tiefen aufweisen.

8. Vorrichtung nach einem der Ansprüche 1 bis 7,
**dadurch gekennzeichnet, dass** sie mindestens ein Längselement, das durch einen einzelnen Stab (36) mit relativ großem Durchmesser gebildet ist, und mindestens ein Längselement umfasst, das durch mehrere Stäbe (41, 42) mit relativ kleinem Durchmesser gebildet ist, und dass das Verbindungsmittel (31) Sitze (35, 40) mit Breiten umfasst, die im Wesentlichen gleich dem Durchmesser der Stäbe sind, die sie enthalten.

9. Vorrichtung nach einem der Ansprüche 1 bis 8,
**dadurch gekennzeichnet, dass** das Verbindungsmittel mindestens einen Stopfen (16) umfasst, der ein Außengewinde (22) aufweist und der in einen Sitz (17) mit einem entsprechenden Gewinde (19) eingesetzt ist, und wobei der Stopfen (16), wenn er festgeschraubt ist, mindestens eines der Längselemente in seinem Sitz hält.

10. Vorrichtung nach Anspruch 9,
**dadurch gekennzeichnet, dass** der Stopfen (16) aus einem oberen Teil (20) mit dem Außengewinde (22) und aus einem unteren Teil hergestellt ist, das durch eine Platte (25) gebildet ist, die über eine Kugel (24) an dem oberen Teil gelenkig angebracht ist.

11. Vorrichtung nach einem der Ansprüche 1 bis 4,
**dadurch gekennzeichnet, dass** die Sitze (11, 12) durch Aussparungen (26, 27), die an der Unterseite des Kopfs (4) des Knochenverankerungselements hergestellt sind, durch einen Gewindevorsprung (28), der an der Unterseite des Kopfs (4) des Knochenverankerungselements hergestellt ist, und durch die untere Seite (30) einer Mutter (29), die durch den Vorsprung (28) getragen ist, gebildet sein können, wobei die Mutter (29) ermöglicht, die Längselemente in den Sitzen (11, 12) zu halten, nachdem sie festgeschraubt wurde.

12. Vorrichtung nach Anspruch 11,
**dadurch gekennzeichnet, dass** der Vorsprung (28) eine Ausnehmung umfassen kann, die es ermöglicht, ihn abzubrechen, nachdem die Mutter (29) festgeschraubt wurde.

## Revendications

1. Dispositif d'ostéosynthèse de la colonne vertébrale, du type comprenant une pluralité d'éléments d'ancrage osseux (2 ; 33, 34), une pluralité d'éléments longitudinaux (5 à 10 ; 36, 41, 42) et des moyens de raccordement (4 ; 31) pour raccorder lesdits éléments longitudinaux (5 à 10 ; 36, 41, 42) auxdits éléments d'ancrage osseux (2 ; 33, 34), dans lequel au moins l'un des éléments longitudinaux est formé d'une pluralité de tiges indépendantes (5 à 7 ; 8 à 10 ; 41, 42) et dans lequel lesdits moyens de raccordement (4 ; 31) comprennent au moins une assise (11, 12 ; 40) présentant une largeur sensiblement égale au diamètre de chaque tige (5 à 10 ; 41, 42), **caractérisé en ce que** lesdites tiges (5 à 10 ; 41, 42) sont empilées dans ladite assise, de façon à former une colonne en coupe.

2. Dispositif selon la revendication 1, **caractérisé en ce qu'**au moins certains desdits moyens de raccordement comprennent les têtes (4) des éléments d'ancrage osseux (2).

3. Dispositif selon la revendication 1 ou 2, **caractérisé en ce qu'**au moins certains desdits moyens de raccordement comprennent des connecteurs (31) formés d'un seul tenant avec lesdits éléments d'ancrage osseux (33, 34).

4. Dispositif selon l'une des revendications 1 à 3, **caractérisé en ce qu'**il comprend au moins deux éléments longitudinaux, chacun formé par une pluralité de tiges (5 à 7, 8 à 10), lesdits moyens de raccordement comprenant des assises (11, 12) présentant une largeur sensiblement égale au diamètre de chaque tige (5 à 7, 8 à 10), en un nombre égal au nombre d'éléments longitudinaux.

5. Dispositif selon l'une des revendications 1 à 4, **caractérisé en ce que** lesdits moyens de raccordement comprennent au moins une ouverture latérale (15) pour l'introduction desdites tiges (5 à 10) dans lesdites assises (11, 12).

6. Dispositif selon la revendication 5, **caractérisé en ce que** ladite ouverture latérale (15) a une largeur de 0,5 à 2 mm supérieure au diamètre de chacune des tiges (5 à 10).

7. Dispositif selon l'une des revendications 4 à 6, **caractérisé en ce que** lesdites assises (11, 12) ont des profondeurs différentes.

8. Dispositif selon l'une des revendications 1 à 7, **caractérisé en ce qu'**il comprend au moins un élément longitudinal formé par une tige simple (36) de diamètre relativement grand et au moins un élément longitudinal formé par une pluralité de tiges (41, 42) de diamètre relativement petit, et **en ce que** lesdits moyens de raccordement (31) comprennent des assises (35, 40) présentant des largeurs sensiblement égales au diamètre des tiges qu'elles contiennent.

9. Dispositif selon l'une des revendications 1 à 8, **caractérisé en ce que** les moyens de raccordement comprennent au moins une fiche (16) qui a un filetage externe (22) et qui est insérée dans une assise (17) ayant un filetage correspondant (19), et ladite fiche (16), lorsqu'elle est serrée, maintient au moins l'un des éléments longitudinaux dans son assise.

10. Dispositif selon la revendication 9, **caractérisé en ce que** ladite fiche (16) est constituée d'une partie supérieure (20) ayant ledit filetage externe (22), et d'une partie inférieure formée par une plaque (25) qui est articulée sur ladite partie supérieure via une bille (24).

11. Dispositif selon l'une des revendications 1 à 4, **caractérisé en ce que** lesdites assises (11, 12) peuvent être formées par des cavités (26, 27) constituées sur le fond de la tête (4) de l'élément d'ancrage osseux, par une proéminence filetée (28) constituée sur le fond de la tête (4) de l'élément d'ancrage osseux, et par la face inférieure (30) d'un écrou (29) porté par la proéminence (28), ledit écrou (29) permettant, après avoir été serré, de maintenir les éléments longitudinaux dans lesdites assises (11, 12).

12. Dispositif selon la revendication 11, **caractérisé en ce que** ladite proéminence (28) peut porter une section qui permet de la casser après que l'écrou (29) a été serré.
